**Europäisches Patentamt**

**European Patent Office**

**Office européen des brevets**

(11) Veröffentlichungsnummer: **0 051 833**
A1

# (12) EUROPÄISCHE PATENTANMELDUNG

(21) Anmeldenummer: **81109308.7**

(22) Anmeldetag: **29.10.81**

(51) Int. Cl.³: **A 61 K 31/685,** A 61 K 45/06, A 61 K 7/48, A 61 K 9/66, A 61 K 9/08

(30) Priorität: **10.11.80 DE 3042365**

(43) Veröffentlichungstag der Anmeldung: **19.05.82** **Patentblatt 82/20**

(84) Benannte Vertragsstaaten: **CH FR GB IT LI NL**

(71) Anmelder: **Extrakta Strauss GmbH, Lokstedter Holt 30, D-2000 Hamburg 61 (DE)**

(72) Erfinder: **Harsanyi, Eugen, Dr., Ulrichstrasse 17, D-5014 Kerpen-Sindorf (DE)**

(74) Vertreter: **Bühling, Gerhard, Dipl.-Chem. et al, Patentanwaltsbüro Tiedtke-Bühling-Kinne Grupe-Pellmann Bavariaring 4, D-8000 München 2 (DE)**

(54) **Flüssige Lecithin-haltige einphasige Mehrstoffsysteme.**

(57) Flüssige Lecithin-haltige einphasige Mehrstoffsysteme enthalten als Bestandteil amphiphile Phospholipide oder Phospholipidgemische sowie Wirkstoffe, welche in den amphiphilen Phospholipiden nicht oder wenig löslich sind. Diese flüssigen lecithinhaltigen einphasigen Mehrstoffsysteme werden dadurch erhalten, daß die Bestandteile dieser Mehrstoffsysteme einzeln oder zu mehreren in einem Lösungsmittel oder Lösungsmittelgemisch gelöst werden, die Lösungen zusammengefügt werden und anschließend die Lösungsmittel entfernt werden. Das flüssige lecithinhaltige einphasige Mehrstoffsystem kann für Arzneimittel verwendet werden.

EP 0 051 833 A1

ACTORUM AG

**Tiedtke - Bühling - Kinne**
**Grupe - Pellmann**

**Patentanwälte** und
**Vertreter beim EPA**
Dipl.-Ing. H. Tiedtke
Dipl.-Chem. G. Bühling
Dipl.-Ing. R. Kinne
Dipl.-Ing. P. Grupe
Dipl.-Ing. B. Pellmann

**Bavariaring 4, Postfach 20 24 03**
**8000 München 2**
Tel.: 0 89 - 53 96 53
Telex: 5-24 845 tipat
cable: Germaniapatent München

- 2 -

**005 1833**

29. Oktober 1981

EP 1594

Extrakta Strauss GmbH

2000 Hamburg 61

Flüssige Lecithin-haltige einphasige Mehrstoffsysteme

Die Erfindung bezieht sich auf flüssige Lecithin-haltige einphasige Mehrstoffsysteme, welche einen oder mehrere in diesen Systemen ansich schwer oder nicht lösliche Bestandteile enthalten, auf diese enthaltende Präparate und auf Verfahren zu deren Herstellung.

Zahlreiche Wirkstoffe sind in ihrer Wirksamkeit dadurch eingeschränkt, daß sie aufgrund ihrer Struktur bzw. Kristallstruktur eine zu kleine Oberfläche haben, wodurch sich ihre Auflösungsgeschwindigkeit verringert. Manche Arzneimittel üben eine unerwünschte Reizwirkung aus, weil sie infolge ihrer kristallinen Struktur die Zellwände beschädigen können. Andere Wirkstoffe bzw. Arzneimittel sollten deswegen in gelöster Form vorliegen, weil sie dann eine visuelle

/13

Kontrolle auf Verunreinigungen ermöglichen, aber auch weil sie in Folge ihrer Transparenz eine bessere Verbraucherakzeptanz finden. Oft ist es aber nicht möglich, eine Lösung dieser Wirkstoffe herzustellen, da die Stoffe in Lösung nicht beständig sind. Dies trifft beispielsweise für wäßrige Lösungen von Penicillin-G-Natrium zu.

Eine Flüssigkeit wird als optisch blank bezeichnet, wenn sie keine Partikel enthält, welche größer als die Lichtwellenlänge sind. Aus Stabilitätsgründen ist es wünschenswert, derartige Wirkstoffe in ein Flüssigkeitssystem einzubringen, welches als wesentliche Komponente einen Stoff enthält, der kaum oder gar nicht Lösungseigenschaften für den Wirkstoff hat. Es ist dabei unerheblich, ob die einphasige Flüssigkeit, welche den Wirkstoff oder die Wirkstoffe enthält, hoch oder niedrigviskos ist. Es ist jedoch wünschenswert, durch Zusatz eines weiteren Bestandteiles die Viskosität auf einen gewünschten Wert einstellen zu können.

Die Wirkstoffe sind häufig hydrophile Stoffe, zu denen auch Salze gehören. Bei diesen Stoffen ist die Einbettung in eine hydrophobe Flüssigkeit erwünscht, da dann insbesondere die Lagerstabilität der Wirkstoffe gesichert erscheint. Neben den hydrophoben Flüssigkeitsbestandteilen sind aber auch amphiphile Stoffe in der Flüssigkeit erwünscht, da diese als Solubilisierhilfsmittel für die Wirkstoffe wirken können.

Zu den hydrophoben Flüssigkeiten können zum Beispiel die Glyzerinester der Fettsäuren gerechnet werden.

Amphiphile Verbindungen sind insbesondere die meisten Phospholipide.

Wiederholt sind schon Versuche unternommen worden, Wirkstoffe in flüssige Systeme einzubringen, um sie dort stabil zu halten.

Die US-Patentschrift 2 832 721 beschreibt ein Verfahren zur Herstellung Lecithin-haltiger Vitaminemulsionen, in welchem eine Lösung von Lecithin in einem organischen Lösungsmittel mit einer Lösung eines Vitamins in einem niederen, aliphatischen Alkohol vereinigt wird, worauf unter Rühren Wasser zugefügt und dann das organische Lösungsmittel unter Vakuum entfernt wird. Die so erhaltene Flüssigkeit ist jedoch optisch nicht blank, so daß ihre einwandfreie Beschaffenheit mittels visueller Kontrolle nicht nachgeprüft werden kann.

Das gleiche Problem tritt bei dem nach dem Verfahren der US-Patentschrift 3 636 194 hergestellten Präparat auf.

Aufgabe der Erfindung sind flüssige, Lecithin-haltige, einphasige Nährstoffsysteme, welche einen oder mehrere Wirkstoffe enthalten und welche dadurch gekennzeichnet sind, daß diese Wirkstoffe an sich in den übrigen Bestandteilen des Systems schwer- oder unlöslich sind.

Die Lösung der Aufgabe besteht in Flüssigkeiten beliebiger Viskosität, welche hydrophobe und amphiphile Bestandteile sowie die im wesentlichen hydrophilen Wirkstoffe enthalten.

Hydrophobe Bestandteile sind vorzugsweise die Glyzerinester der Fettsäuren sowie deren Derivate, die beispielsweise durch Polyäthoxilierung oder Acylierung erhalten werden sowie weitere nicht-ionogene hydrophobe Stoffe, wie zum Beispiel Polyätherglykole. Diese hydrophoben Bestandteile sollen im wesentlichen flüssig sein, müssen jedoch, insbesondere wenn sie nicht molekular einheitlich sind, nicht ausschließlich aus flüssigen Anteilen bestehen.

Zu den amphiphilen Komponenten der flüssigen Lecithin-haltigen einphasigen Mehrstoffsysteme zählen insbesondere die Phospholipide, wobei Phospholipidgemische bevorzugt werden. Besonders bevorzugt werden dabei Phospholipidgemische mit einem Gehalt an Phosphatidylcholin von 20 bis 80 %, davon wieder insbesondere 30 bis 60 %, und einem Gehalt von Phosphatidyläthanolamin von 5 bis 40 %, vorzugsweise 10 bis 20 %. Unter den Phospholipidgemischen werden diejenigen bevorzugt, welche in organischen Lösungsmitteln mit Dielektrizitätskonstanten (DK) zwischen 3 und 50, vorzugsweise zwischen 5 und 35, löslich sind.

Als Wirkstoffe kommt eine große Zahl von Stoffen aus unterschiedlichen Stoffklassen in Betracht, sofern sie nur die Bedingung erfüllen, in den hydrophoben oder amphiphilen Bestandteilen der Mehrstoffsysteme unlöslich oder weniglöslich zu sein. Diese Bedingung wird beispielsweise von folgenden Wirkstoffen erfüllt: Ascorbinsäure, Sterine wie Brassicasterin, Cholesterin und ß-Sitosterin, Cytostatika wie Colchicinderivate, Acetylsalicylsäure und Natriumsalicylat, Penizillin-G-Natrium und -Kalium, Sulfonamide wie zum Beispiel Sulfadiazin oder Sulfadimidin.

Während der Mengenanteil der hydrophoben Mischungsbestandteile, welche als Viskositätskorrigenz dienen, nur von der gewünschten Viskosität bestimmt wird, ist es wichtig, beim Mengenverhältnis zwischen den amphiphilen Phospholipiden und den Wirkstoffen Grenzen einzuhalten. Auf die Gewichte bezogen, sollen die Wirkstoffe zwischen 0,5 und 80 % der Menge der amphiphilen Phospholipide betragen, vorzugsweise maximal 60 % und noch weiter bevorzugt maximal 50 %.

Die Herstellung der flüssigen Lecithin-haltigen einphasigen Mehrstoffsysteme erfolgt in einer besonderen Ausgestaltung der Erfindung dadurch, daß man die Bestandteile der genannten Mehrstoffsysteme in Lösungsmitteln, welche intermediär als Hilfsstoffe dienen, löst, die Lösungen vereinigt und die als Hilfsstoffe verwendeten Lösungsmittel wieder entfernt, was vorzugsweise unter Abdampfen im Vakuum geschehen kann. Es ist dabei möglich, daß beim Zusammenfügen der Lösungen zunächst Niederschläge auftreten, welche aber nach der Entfernung der Lösungsmittel wieder verschwunden sein müssen. In einer Ausführungsform der Erfindung wird eine Lösung aus dem Phospholipidgemisch und dem hydrophoben Viskositätskorrigenz hergestellt, welche dann mit einer separat hergestellten Lösung des oder der Wirkstoffe vermischt wird, wonach die als Hilfsstoffe dienenden Lösemittel entfernt werden. Es ist möglich, für alle Bestandteile des Mehrstoffsystems das gleiche Lösungsmittel zu verwenden, jedoch können für die einzelnen Bestandteile des Mehrstoffsystems auch unterschiedliche Lösemittel eingesetzt werden. Es ist dabei nicht unbedingt notwendig, daß in jedem der verwendeten Lösemittel alle Bestandteile des Mehrstoffsystems löslich wären. Beispielsweise können die Wirkstoffe in Wasser oder einem wasserhaltigen Lösemittel gelöst werden, während die Phospholipide und gegebenenfalls die Viskositätskorrigenzien in Äthanol gelöst werden.

Die Erfindung wird durch die nachstehenden Beispiele näher erläutert.

Beispiel 1

5 g eines alkohollöslichen Gemisches aus Sojaphospholipiden, welches 46 % Phosphatidylcholin, 23 % Phosphatidyläthanolamin und 1 % Phosphatidylinosit enthält, werden gemeinsam mit 5 g eines durch Veresterung von Glyzerin mit Kokosvorlaufsäuren erhaltenen Öles in 70 ml absolutem Äthanol gelöst. Zu dieser Lösung wird eine Lösung von 520 mg Penicillin-G-Kalium in 2,5 ml entionisiertem Wasser vorsichtig hinzugefügt, wobei eine Trübung entsteht. Nun werden das Wasser und das Äthanol unter intensivem Rühren bei 42$^\circ$C im Vakuum abgedampft. Es entsteht eine optisch blanke ölige Flüssigkeit.

Beispiel 2

Es wurde wie in Beispiel 1 verfahren, jedoch wurde anstelle von Penizillin-G-Kalium Penizillin-G-Natrium verwendet. Auch hier entstand eine optisch blanke ölige Flüssigkeit.

Beispiel 3

Es wurde wie in Beispiel 1 verfahren, jedoch wurde die Menge an Penizillin-G-Kalium auf 1,2 g und die Menge an Wasser auf 4 ml erhöht. Es wurde wiederum eine optisch blanke Flüssigkeit erhalten, deren Viskosität jedoch deutlich höher als im Beispiel 1 war.

Beispiel 4

Es wurde wie in Beispiel 1 verfahren, jedoch wurde statt der alkohollöslichen Mischung aus Sojaphospholipiden unfraktioniertes entöltes Sojalecithin in einer Menge von 6 g eingesetzt.

Es wurde eine optisch blanke ölige Flüssigkeit erhalten.

Beispiel 5

Es wurde wie in Beispiel 1 verfahren, jedoch wurde statt des Penicillin-G-Kaliums Acetylsalicylsäure verwendet. Es wurde eine optisch blanke ölige Flüssigkeit erhalten.

Beispiel 6

5 g Ascorbinsäure wurden in 25 ml Wasser aufgelöst. Die Lösung wurde zu einer Lösung von 95 g nichtentöltem Sojalecithin mit einem Gehalt von 61 % acetonunlöslichen Bestandteilen in 500 ml Äthanol gegeben. Nach vorsichtigem Abdampfen des Wassers und des Äthanols wurde eine optisch blanke ölige Flüssigkeit erhalten.

Beispiel 7

500 mg Sulfadiazin wurden in 8 ml Dimethylformamid aufgelöst. Diese Lösung wurde zu einer Lösung von 7 g eines Gemisches aus Sojaphospholipiden mit einem Gehalt an 62 % Phosphatidylcholin, 19 % Phosphatidyl-äthanolamin und 3 % Phosphatidylinosit in 48 ml

0051833

Dimethylformamid hinzugefügt. Nach vorsichtigem Abdampfen des Dimethylformamids unter Vakuum bei 48°C wurde eine optisch blanke ölige Flüssigkeit erhalten.

Beispiel 8

Es wurde wie in Beispiel 7 verfahren, jedoch wurde den beiden Lösungen eine weitere Lösung von 2 g Glyzerin-Monooleat in 10 ml Äthanol hinzugefügt. Nach Abziehen der Lösemittel wurde eine optisch blanke ölige Flüssigkeit mit geringerer Viskosität als die in Beispiel 7 erhalten.

Beispiel 9

Es wurde verfahren wie in Beispiel 1, jedoch wurde statt des Phospholipidgemisches ein zu 98 % reines Phosphatidylcholin, das aus Sojalecithin hergestellt worden war, verwendet. Nach Abdampfen der Lösemittel wurde eine Flüssigkeit erhalten, in der der größte Teil des Wirkstoffes als Niederschlag ausgefallen war.

Beispiel 10

Es wurde wie in Beispiel 1 verfahren, jedoch wurde ein aus Eilecithin erhaltenes Phospholipidgemisch mit einem Gehalt an 85 % Phosphatidylcholin verwendet. Es wurde ein Ergebnis wie in Beispiel 9 erhalten.

Patentanwälte und
Vertreter beim-EPA
Dipl.-Ing. H. Tiedtke
Dipl.-Chem. S. Bühling
Dipl.-Ing. R. Kinne
Dipl.-Ing. P. Grupe
Dipl.-Ing. B. Pellmann

**Bavariaring 4, Postfach 20 24 03**
**8000 München 2**
Tel.: 0 89 - 53 96 53
Telex: 5-24 845 tipat
cable: Germaniapatent München

29. Oktober 1981

EP 1594

00 51 833

Patentansprüche

1. Flüssige Lecithin-haltige einphasige Mehrstoffsysteme, dadurch gekennzeichnet, daß sie als Bestandteile amphiphile Phospholipide oder Phospholipidgemische sowie Wirkstoffe enthalten, welche in den amphiphilen Phospholipiden nicht oder wenig löslich sind.

2. Flüssige Lecithin-haltige einphasige Mehrstoffsysteme nach Anspruch 1, dadurch gekennzeichnet, daß sie zusätzlich hydrophobe Bestandteile enthalten, welche als Viskositätskorrigentien wirksam sind.

3. Flüssige Lecithin-haltige einphasige Mehrstoffsysteme nach Anspruch 1 oder 2, dadurch gekennzeichnet, daß die gewichtsmäßigen Anteile der Wirkstoffe 0,5 bis 80 %, vorzugsweise 0,5 bis 60 % und bsonders bevorzugt 0,5 bis 40 % der Anteile der amphiphilen Phospholipide betragen.

4. Flüssige Lecithin-haltige einphasige Mehrstoffsysteme nach einem der Ansprüche 1 bis 3, dadurch gekennzeichnet, daß die amphiphilen Phospholipidge-

COMPLETE DOCUMENT

/13

mische zu 20 bis 80 % aus Phosphatidylcholin, 5 bis 40 % aus Phosphatidyläthanolamin und Rest, vorzugsweise 0,5 bis 10 % aus Phosphatidylinosit bestehen.

5. Flüssige Lecithin-haltige einphasige Mehrstoffsysteme nach einem der vorhergehenden Ansprüche, dadurch gekennzeichnet, daß sie als Wirkstoffe Penizillin-G-Natrium oder -Kalium, Ascorbinsäure, Sterine wie Brassicasterin, Cholesterin oder ß-Sitosterin, Cytostatika wie Colchicinderivate, Acetylsalicylsäure, Natriumsalicylat, Sulfonamide wie Sulfadiacin oder Sulfadimidin oder Mischungen der genannten Wirkstoffe oder weitere Wirkstoffe oder Mischungen der genannten Wirkstoffe mit weiteren Wirkstoffen enthalten.

6. Flüssige Lecithin-haltige einphasige Mehrstoffsysteme nach einem der vorhergehenden Ansprüche, dadurch gekennzeichnet, daß sie als Viskositätskorrigentien Glyzerinester von Fettsäuren und/oder acylierte und/oder methoxylierte Derivate dieser Glyzerinester oder ähnlich gebaute Verbindungen enthalten.

7. Flüssige Lecithin-haltige einphasige Mehrstoffsysteme nach einem der vorhergehenden Ansprüche, dadurch gekennzeichnet, daß die Flüssigkeit in peroral anwendbaren Kapseln abgefüllt ist, wobei diese Kapseln vorzugsweise durchsichtig sind.

8. Verfahren zur Herstellung flüssiger Lecithinhaltiger einphasiger Mehrstoffsysteme nach einem der Ansprüche 1 bis 8, dadurch gekennzeichnet, daß die Bestandteile dieser Mehrstoffsysteme einzeln oder zu mehreren in einem Lösungsmittel oder Lösemittelgemisch gelöst werden, die Lösungen zusammengefügt werden und anschließend die Lösemittel entfernt werden.

9. Verfahren nach Anspruch 8, dadurch gekennzeichnet, daß zum Auflösen der amphiphilen Phospholipide Lösemittel mit einer Dielektrizitätskonstante von 3 bis 50, vorzugsweise von 5 bis 35 verwendet werden.

10. Arzneimittel, dadurch gekennzeichnet, daß es mindestens einen pharmakologischen Wirkstoff in einem flüssigen Lecithin-haltigen einphasigen Mehrstoffsystem nach einem der vorhergehenden Ansprüche neben üblichen pharmazeutisch verträglichen Zusätzen enthält.

Europäisches
Patentamt

**EUROPÄISCHER RECHERCHENBERICHT**

0051833

Nummer der Anmeldung

EP 81 10 9308

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | **A 61 K 31/685** 45/06 7/48 9/66 9/08 |
| X | CHEMICAL ABSTRACTS, Band 34, Nr. 21, 10. November 1940, Zusammenfassung 7529-7 COLUMBUS, OHIO (US) A. MOSSINI et al.: "Preparation of lecithin solutions for injections" & Boll. chim.-farm. 79, 177-8 (1940) | | |
| | * insgesamt * --- | 1-10 | |
| X | DE - C - 501 502 (CHEMISCH-PHARMA-ZEUTISCHE A.G.) | | **RECHERCHIERTE SACHGEBIETE (Int. Cl.³)** |
| | * Seite 1, Zeile 35 bis Seite 2, Zeile 32; Beispiele 1-4 * --- | 1-10 | **A 61 K** |
| X | DE - C - 663 512 (C.H. BUER) | | |
| | * Seite 2, Zeilen 20-28; Patentansprüche 1 und 2 * --- | 1-10 | |
| X | FR - A - 1 519 516 (MINISTERUL INDUSTRIEI PETROLULUI SI CHIMEI) | | |
| | * Seite 1, rechte Spalte, letzter Absatz bis Seite 2, linke Spalte, letzter Absatz; Beispiel 1 * & DE - C - 1 595 936 --- | 1-10 | |
| X | FR - A - 2 453 644 (A. NATTERMANN & CIE. GmbH) | | **KATEGORIE DER GENANNTEN DOKUMENTE** |
| | * Seite 8, Zeilen 1-30; Ansprüche 1-5 * & DE - A - 2 914 788 --- ./.. | 1-10 | X: von besonderer Bedeutung allein betrachtet Y: von besonderer Bedeutung in Verbindung mit einer anderen Veröffentlichung derselben Kategorie A: technologischer Hintergrund O: nichtschriftliche Offenbarung P: Zwischenliteratur T: der Erfindung zugrunde liegende Theorien oder Grundsätze E: älteres Patentdokument, das jedoch erst am oder nach dem Anmeldedatum veröffentlicht worden ist D: in der Anmeldung angeführtes Dokument L: aus andern Gründen angeführtes Dokument |

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 12.02.1982 | BRINKMANN |

EPA form 1503.1  06.78

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | FR - A - 2 453 646 (A. NATTERMANN & CIE. GmbH) <br><br> * Seite 11, Zeilen 1-34; Ansprüche 1-7 * <br><br> && DE - A - 2 914 789 <br> --- | 1-10 | |
| X | UNLISTED DRUGS, Band 28, Nr. 1, Januar 1976, Seite 12, CHATHAM, N.J. (US) <br><br> * Seite 12k: "Trive 1000" * <br> --- | 1-10 | |
| X | UNLISTED DRUGS, Band 14, Nr. 4, April 1962, Seite 36, CHATHAM, N.J. (US) <br><br> * Seite 36k: "Dantrol" * <br> --- | 1-10 | RECHERCHIERTE SACHGEBIETE (Int. Cl.³) |
| X | ROTE LISTE, 2. März 1961, EDITIO CANTOR, AULENDORF/WÜRTT. (DE) <br><br> * Seite 325: "Essentiale Ampullen"; Seite 858: "Dr. Sidler Bionervin" und "Dr. Sidler Brom-Bionervin"; Seite 119, "Biocitin-Flüssig"* <br> --- | 1-10 | |
| P/X | WO - A - 81/02673 (KEY PHARMACEUTICALS INC.) <br><br> * Seite 27, Ansprüche 1-7 * <br> --- | 1-10 | |
| D/A | US - A - 3 636 194 (DOUGLAS G. PARIZEAU) | 1-10 | |
| D/A | US - A - 2 832 721 (R.R. DEGKWITZ) | | |

------------